# EUROPÄISCHE PATENTANMELDUNG

(11) **EP 2 196 802 A2**
(43) Veröffentlichungstag der Anmeldung: **16.06.2010**
(21) Anmeldenummer: 09174339.3
(22) Anmeldetag: 28.10.2009
(51) Int. Cl.: G01N 33/34

(54) **Automatisierungssystem**

(30) Priorität: 04.12.2008 DE 102008044343
(71) Anmelder: Voith Patent GmbH, 89522 Heidenheim (DE)
(72) Erfinder: Münch, Rudolf, 89551 Königsbronn (DE)

(57) **Zusammenfassung**

Erfindungsgemäß wird eine Vorrichtung zur Aufnahme von Messdaten in einer Maschine zur Herstellung von Papier und/oder Tissue und/oder Karton und/oder Verpackungspapier vorgeschlagen, die mind. ein umschlossenes Teilsystem mit einer Umschliessung (25) aufweist. Wobei dem Teilsystem ein Knotenpunkt (3) mit mind. einem angeschlossener Sensor und/oder Aktor zugeordnet ist. Zudem wird das Teilsystem durch mind. ein System mit Prozessenergieenergie (27) versorgt wie z.B. Wasserdampf, Erdgas, Abgas oder anderen Energieträgern. Des Weiteren ist eine Verbindung zu einem Leitsystem (7) und/oder anderen Teilsystemen vorhanden.

Die Verbindung des Knotenpunktes (3) zum Leitsystem und/oder einem anderen Knotenpunkt ist erfindungsgemäß eine Drahtlose Verbindung (6) und zur Energieversorgung des Knotenpunktes mit den angeschlossenen Sensoren und/oder Aktoren (5) ist ein separates Energieerzeugungssystem (4) vorhanden.

## Beschreibung

Die Erfindung betrifft eine Maschine zur Herstellung von Papier und/oder Tissue und/oder Karton und/oder Verpackungspapier mit mindestens einem umschlossenen Teilsystem und mindestens einem System zum Zu- und Abführen von Energie.

Eine Papiererzeugungsanlage besteht aus vielen Teilsystemen, die in einem komplexem System mit einem Leitsystem verbunden sind, so dass Prozessdaten aller Teilsysteme zusammengeführt werden können und so der komplexe Gesamtprozess gesteuert/geregelt und überwacht werden kann.
Nur wenn alle bzw. möglichst viele Daten bekannt sind, kann der Gesamtprozess optimiert werden.

Da aber viele Teilsysteme in einer Papiermaschine auf einem erhöhten Temperaturniveau betrieben werden und zudem noch von einem Gehäuse umschlossen sind und sich drehen, wie zum Beispiel eine Trockenzylinder, ist es sehr aufwendig alle benötigten Prozessdaten aus solchen Teilsystemen zu erhalten.

Die Trockenpartie einer Papiermaschine z.B. enthält eine Vielzahl von Walzen, die jeweils ein Teilsystem bilden. An jeder Walze, oder auch am Lagergehäuse der Walze, ist für Überwachungszwecke mindestens ein Sensor, z.B. ein Beschleunigungs- und/oder Temperatursensor angebracht. Aufgrund der Bedingungen im Bereich eines Trockenzylinders erfolgt der Anschluss dieser Sensoren mittels stationärer Messleitungen mit einer äußerst aufwendigen Kabelführung, an das Leitsystem. Sensoren innerhalb von Walzen werden nach dem Stand der Technik in der Regel vermieden, obwohl sie wertvolle Informationen zur Optimierung des Gesamtsystems bieten würden.

Bisher gibt es keine Lösung die Signale zur Datenübertragung wie auch die Energie für die Sensoren kabellos zur Verfügung zu stellen, um so beispielsweis zu einer Trockenpartie (18) den Verkabelungsaufwand zu vermeiden.

Aus dem Stand der Technik sind zwar verschiedene kabellose Datenübertragungssysteme für allerlei Anwendungsgebiete bekannt, aber besonders problematisch ist die gleichzeitige kabellose Energieversorgung eines solchen Systems, insbesondere, da Lösungen mit Batterien nicht erwünscht sind. Batterien sind nicht sehr zuverlässig und müssen häufig gewechselt werden.

Der Erfindung liegt daher die Aufgabe zugrunde, ein Messvorrichtung sowie eine Verfahren zum kostengünstigen und störungsarmen Erfassung und Übertragung von Mess- und Prozessdaten an ein Leitsystem in einer Maschine zur Herstellung von Papier und/oder Tissue und/oder Karton und/oder Verpackungspapier aufzuzeigen.

Die Aufgabe wird mittels einer Vorrichtung mit den Merkmalen des Anspruchs 1 bzw. mittels eines Verfahrens mit den Merkmalen des Anspruches 9 gelöst.

Erfindungsgemäß wird eine Vorrichtung zur Aufnahme von Messdaten in einer Maschine zur Herstellung von Papier und/oder Tissue und/oder Karton und/oder Verpackungspapier vorgeschlagen, die mindestens ein umschlossenes Teilsystem aufweist. Wobei dem Teilsystem ein Knotenpunkt mit mindestens einem angeschlossener Sensor und/oder Aktor zugeordnet ist. Zudem wird das Teilsystem durch mindestens ein System mit Prozessenergieenergie versorgt wie z.B. Wasserdampf, Erdgas, Abgas oder eines Kühlmediums. Des Weiteren ist eine Verbindung zu einem Leitsystem und/oder anderen Teilsystemen vorhanden.
Wobei die Verbindung des Knotenpunktes zum Leitsystem und/oder einem anderen Knotenpunkt ist erfindungsgemäß eine drahtlose Verbindung ist und zur Energieversorgung des Knotenpunktes mit den angeschlossenen Sensoren und/oder Aktoren ein lokales Energieerzeugungssystem vorhanden ist.

Die drahtlose Verbindung ist vorzugsweise eine WLAN Verbindung, aber auch Verbindungen mittels Mikrowellen oder anderen elektromagnetischen Wellen sind denkbar.

Aufgrund der thermischen Bedingungen in einem Teilsystem, wie beispielsweise einer Trockenwalze, wird die Energie für den Knotenpunkt mit den angeschlossenen Sensoren und/oder Aktoren vorteilhaft durch einen Thermoelektrischen (TEG) Generator gewonnen.

Diese sind in der Lage die Temperaturdifferenz zwischen der Prozessenergie und einem von der Prozessenergie abgeschotteten kühleren Bereich zur Energieerzeugung zu nützen. Deshalb wird der Thermoelektrische Generator vorzugsweise in der Außenhülle des Teilsystems untergebracht.

Dies hat den weiteren Vorteil, dass der Knotenpunkt des Leitsystems so angeordnet werden kann, dass er für Servicezwecke von außen leicht zugänglich ist. Zudem lässt sich die Elektronik des Knotenpunktes leichter Kühlen, wenn dies nötig sein sollte, als wenn sie sich im Inneren des Gehäuses - also im Bereich der Prozessenergie - befände.

Der Thermoelektrischen Generator, der Knotenpunkt und die Sensoren und/oder Aktoren bilden eine Einheit, ein Automatisierungssystem. Dabei können die einzelnen Bauteile zusammen in einer Baueinheit untergebracht oder getrennt und mittels Leitungen miteinander verbunden sein. Dies hätte einerseits den Vorteil, dass die einzelnen Bauteile an unterschiedlichen Orten untergebracht sein können, entsprechend ihrer Funktion, und andererseits diese Komponenten so angeordnet werden können, dass die erforderlichen Kabellängen sehr kurz sind. Die üblicherweise längeren Kabelwege vom Knoten zu anderen Elementen des Leitsystems werden auf jeden Fall vermieden.

Auch ist es zur weiteren Optimierung der notwendigen Verkabelung denkbar, dass mehrere unabhängige Automatisierungssysteme mit jeweiligen Knoten in einem Teilsystem angeordnet sind, um möglichst unaufwändig alle gewünschten Prozessdaten zu erhalten

### Figurenbeschreibung

Weitere Merkmale und Vorteile der Erfindung ergeben sich aus der nachfolgenden Beschreibung und des bevorzugten Ausführungsbeispieles unter Bezugnahme auf die Zeichnung.
- Figur 1:: ein prinzipieller Aufbau einer Papierherstellungsmaschine mit Stoffauflauf, Siebpartie, Pressenpartie, Trockenpartie, Kalander und Wickelvorrichtung in Seitenansicht;
- Figur 2:: eine Skizze des erfindungsgemäßen Automatisierungssystems
- Figur 3:: eine Skizze mit einem erweiterten Gesamtsystem

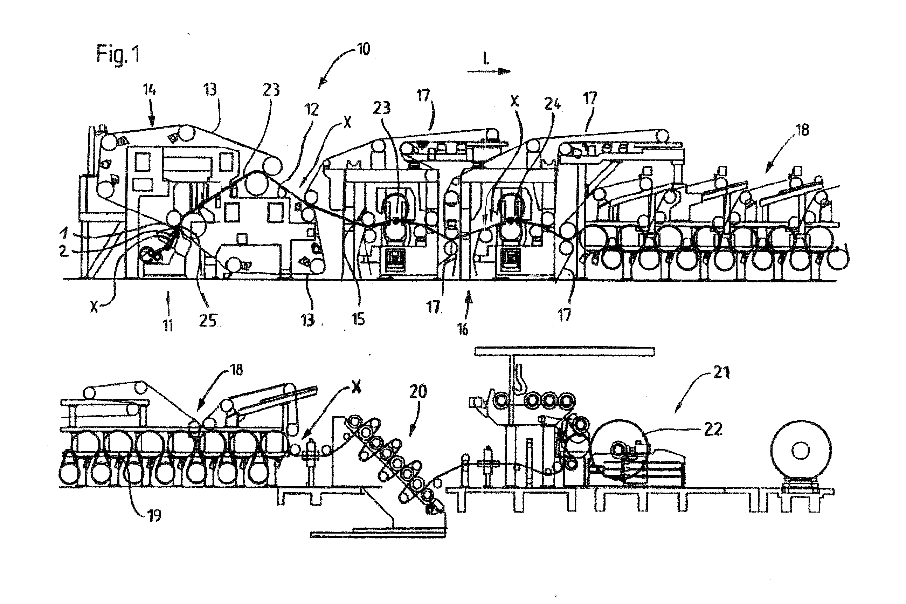

Die Fig. 1 zeigt in Seitenansicht den prinzipiellen Aufbau einer Papierherstellungsmaschine zur Herstellung einer Faserstoffbahn. Die dargestellte Papierherstellungsmaschine weist einen Stoffauflauf zum Einbringen mindestens einer maschinenbreiten Schicht einer Faserstoffsuspension zwischen zwei laufende Siebe 13 einer Siebpartie 14 auf. Die in der Siebpartie 14 formierte und entwässerte Schicht (Faserstoffbahn) 12 wird als nasse Faserstoffbahn 15 an eine Pressenpartie 16, bestehend aus zwei vorzugsweise doppelt befilzten (Langspalt-)Pressen 16, die jeweils einen in Bahnlaufrichtung L (Pfeil) verlängerten Pressspalt 23, 24 bilden, übergeben und dort zwischen den Pressfilzen 17 weiter entwässert. Anschließend wird die feuchte Faserstoffbahn 15 in eine Trockenpartie 18 mit in einer Reihe nebeneinander liegenden Trockenzylindern 19 überführt und unter neanderförmigen Lauf um die beheizten Trockenzylinder 19 getrocknet. Nach der Trockenpartie 18 wird die Faserstoffbahn 15, nunmehr bereits eine trockene Faserstoffbahn, durch einen Kalander 20 zu einer Wickelvorrichtung 21 geleitet und dort zu einer Wickelrolle 22 aufgewickelt.

So stellt z.B. jeder Trockenzylinder ein Teilsystem im Sinne der Erfindung dar. Ein anderes - grösseres - Teilsystem stellt die Trockenpartie 18 dar, die von einer Haube umschlossen wird.

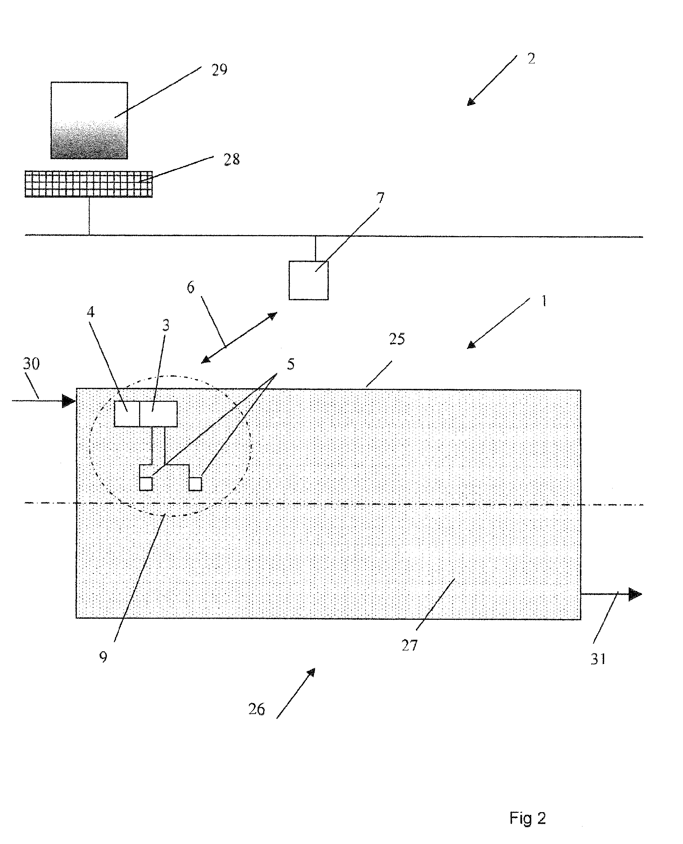

In Fig. 2 ist eine Prinzipskizze der Erfindung dargestellt. Es wird dargestellt wie ein Teilsystem 1 in das Gesamtsystem 2 eingebunden ist. Bei dem Teilsystemen 1 handelt es sich z.B. um eine Trockenpartie 18, deren Walzen 26 mit bis zu 180°C heißem Dampf 27 beheizt werden. In diesem Fall ist also Dampf der Träger für die Prozessenergie.

In dem Teilsystem 1 ist ein Knotenpunkt 3 angeordnet der mit den Sensoren 5 mittels Kabeln verbunden ist. Dabei kann es sich z.B. um Beschleunigungs- und/oder Temperatursensoren handeln. Es könnten aber auch Aktoren angesteuert werden.

Ein Knotenpunkt 3 beinhaltet erfindungsgemäß einen Rechner für die Datenauswertung und Ansteuerung der Komponenten des Automatisierungssystems 9 wie der Sensoren 3 und ein Kommunikationsmittel für die Verbindung zum Gesamtsystem 2 bzw. zum Leitsystem 7. Die Verbindung zum Leitsystem 7 erfolgt erfindungsgemäß über eine Funkverbindung wie z.B. WLAN 6.

Zudem kann der Knotenpunkt 3 noch Kommunikationsmittel enthalten die eine Verbindung zu den anderen Komponenten desselben Automatisierungssystems 9 ermöglichen und optional Mittel um Energie an die anderen Komponenten dieses Automatisierungssystems 9 weiterzugeben.

Der Knotenpunkt 3 es Automatisierungssystems 9 ist gemeinsam mit einer Energiegewinnungsanlage nahe der Umschließung 25 des Teilsystems 1 angebracht, wie z.B. direkt in der Hülle der Trockenpartie 18. Für einen Thermoelektrischen Generator (TEG) 4 als Energiegewinnungsanlage ist an dieser Position ist die Temperaturdifferenz zwischen dem Inneren des Teilsystems 1 und der Umgebung am größten und so zur Energiegewinnung für das Automatisierungssystem 9 einfach nutzbar. Zudem steht auch eine genügend große Fläche zur Energiegewinnung zur Verfügung.

Thermoelektrische Wandler beruhen auf dem Seebeck-Effekt. Mit heutigen TEG ist eine Energieausbeute von ca. 10 W, bei einer Modulfläche von 40x40 mm und einer Differenztemperatur von 100°C, erreichbar. Allerdings ist dann eine zusätzliche Wasserkühlung zur Aufrechterhaltung der Differenztemperatur erforderlich, die z.B. von der Motorkühlung abgezweigt werden kann. Module mit einer geringeren Leistung von 1-2 W pro Modul können auch mit Konvektion betrieben werden. Da an der Außenhülle 25 der Umschließung des Teilsystems der Anlage genügend Platz für mehrere Module zur Verfügung steht und eine Leistung von ca. 100 W für ein Automatisierungssystems 9 typischerweise ausreichen, ist dieses System vorzuziehen.

Derartige Automatisierungssysteme 9 sind besonders geeignet für Monitoringsysteme, da diese beim Stillstand der Maschine nicht benötigt werden. So ist es im Normalfall auch nicht notwendig diese im Stillstand mit Energie zu versorgen. Alternativ kann ein Energiespeicher wie eine aufladbare Spannungsquelle, z.B. ein Akku oder ein Kondensator vorgesehen werden.

Die Sensoren 5 in einem Monitoringsystem benötigen relativ wenig Strom und die Datenverbindung zum Leitsystem ist nur ab und zu notwendig - was den Stromverbrauch für die Datenverbindung zum Leitsystem weiter senkt.

Zur weiteren Reduzierung des Energieverbrauchs ist es auch denkbar, dass mit relativ geringen Ladeströmen ein Energiespeicher aufgeladen wird, der zyklisch alle paar Minuten die Durchführung von Messungen ermöglicht.

Idealerweise wird das Monitoringsystem in Automatisierungssystems mit je 10 bis 30 Sensoren gruppiert, die unabhängig voneinander mit dem Leitsystem kommunizieren. Dieser dezentrale Aufbau reduziert den Aufwand für die Ansteuerung der Sensoren 5 durch den Knotenpunkt 3.

Alternativ kann die Verbindung der Knotenpunkte 3 zu den Sensoren/Aktoren 5 auch kabellos erfolgen, wobei die Sensoren/Aktoren 5 dann selbst in der Lage sein müssen Energie in ihrer direkten Umgebung abzugreifen.

Erfindungsgemäß kann die Energieerzeugung auch aus anderen Arten erfolgen, wie aus Vibration, Schall, Bewegung und Magnetfeldänderung und diese Energie für ihre Stromversorgung zu nützen.

Da Aktuatoren in der Regel große Energiemengen benötigen sind diese nur begrenzt einsetzbar.

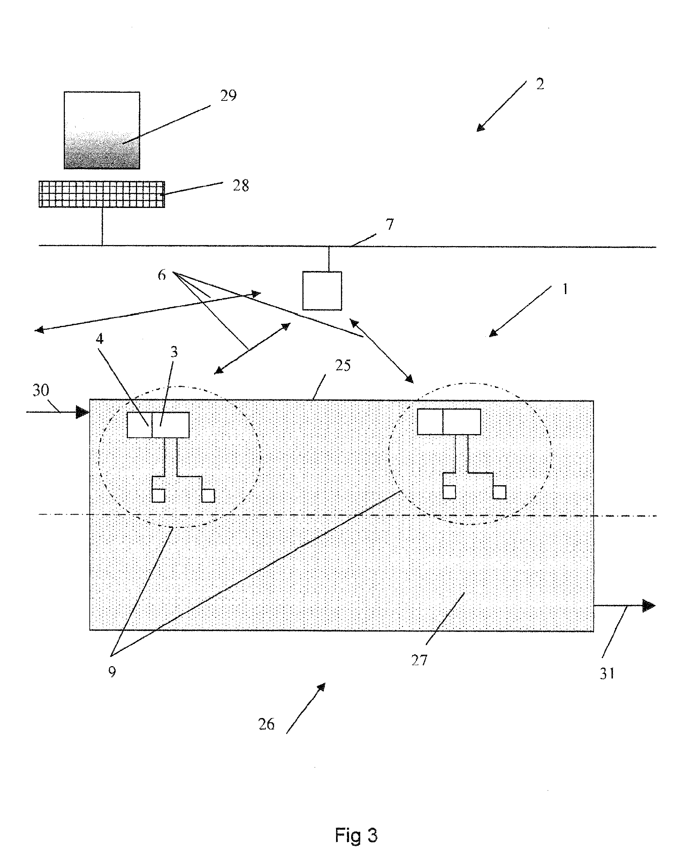

In Fig. 3 ist ein erweitertes Gesamtsystem 2 dargestellt. Es zeigt, dass ein Teilsystem auch mehrere Automatisierungssysteme 9 enthalten kann die unabhängig voneinander arbeiten, aber auch untereinander kommunizieren 6.

### Bezugszeichenliste

- 1: Teilsystem
- 2: Gesamtsystem
- 3: Knotenpunkt
- 4: Thermoelektrischer Generator
- 5: Sensor / Aktor
- 6: drahtlose Verbindung
- 7: Leitsystem
- 8: Datennetzwerk
- 9: Automatisierungssystem
- 10: Vorrichtung (Papierherstellungsmaschine)
- 11: Stoffauflauf (maschinenbreit)
- 12: Faserstoffbahn
- 13: Sieb
- 14: Siebpartie
- 16: Pressenpartie
- 17: Pressfilze
- 18: Trockenpartie
- 19: Trockenzylinder
- 20: Kalander
- 21: Wickelvorrichtung
- 22: Wickelrolle
- 23/24: Pressspalt
- 25: Umschließung/ Außenhülle
- 26: Umgebung der Umschließung
- 27: Prozessenergie, beispielsweise Dampf
- 28: Bedieneinheit
- 29: Bildschirm
- 30: Prozessenergie zufuhr
- 31: Prozessenergie abfuhr

## Patentansprüche

1. Vorrichtung zur Aufnahme von Messdaten in einer Maschine zur Herstellung von Papier und/oder Tissue und/oder Karton und/oder Verpackungspapier mit mindestens einem umschlossenen Teilsystem (1), mindestens einem dem Teilsystem zugeordneten Knotenpunkt (3) mit mindestens einem angeschlossenen Sensor und/oder Aktor (5) sowie einer Verbindung zu einem Leitsystem (7)
**dadurch gekennzeichnet, dass**
für die Verbindung des Knotenpunktes (3) zum Leitsystem (7) und/oder einem anderen Knotenpunkt (3) eine drahtlose Verbindung (6) vorhanden ist und das für die Energieversorgung des Knotenpunktes (3) mit den angeschlossenen Sensoren und/oder Aktoren (5) ein separates Energieerzeugungssystems (4) vorhanden ist.

2. Vorrichtung nach Anspruch 1,
**dadurch gekennzeichnet,**
**dass** die drahtlose Verbindung (6) eine Funk-Verbindung zur Datenübertragung ist.

3. Vorrichtung nach Anspruch 1 oder 2,
**dadurch gekennzeichnet,**
**dass** das Erzeugungssystems (4) ein Thermoelektrischen Generator ist.

4. Vorrichtung nach Anspruch 3,
**dadurch gekennzeichnet,**
**dass** der Thermoelektrischen Generator (4) die Temperaturdifferenz zwischen der dem von der Prozessenergieenergie (27) erwärmten Bereich und einem von der Prozessenergieenergie abgeschotteten kühlerem Bereich zur Energieerzeugung nutzt.

5. Vorrichtung nach einem der vorhergehenden Ansprüche,
**dadurch gekennzeichnet,**
**dass** der Thermoelektrischen Generator (4) in der Außenhülle (25) des Teilsystems (1) untergebracht ist.

6. Vorrichtung nach einem der vorhergehenden Ansprüche,
**dadurch gekennzeichnet,**
**dass** der Thermoelektrischen Generator (4), der Knotenpunkt (3) und die Sensoren und/oder Aktoren (5) eine Einheit, ein Automatisierungssystem (9), bilden.

7. Vorrichtung nach einem der vorhergehenden Ansprüche,
**dadurch gekennzeichnet,**
**dass** mehrere Automatisierungssysteme (9) in einem Teilsystem (1) angeordnet sind.

8. Vorrichtung nach einem der vorhergehenden Ansprüche,
**dadurch gekennzeichnet,**
**dass** die Automatisierungssysteme (9) von außen leicht zugänglich sind.

9. Verfahren zur Aufnahme von Messdaten in einer Maschinen zur Herstellung von Papier und/oder Tissue und/oder Karton und/oder Verpackungspapier mit mind. einem umschlossenen Teilsystem (1), mit mindestens einem dem Teilsystem zugeordneten Knotenpunkt (3) mit angeschlossenen Sensor und/oder Aktor (5), sowie einer Verbindung zu einem Leitsystem (7)
**dadurch gekennzeichnet, dass**
die Verbindung von dem mind. einem Teilsysteme (1) zum Leitsystem (7) und/oder einem anderen Teilsystemen über eine drahtlose Verbindung (6) und
die Energieversorgung der Sensoren und/oder Aktoren mittels eines separaten Energieerzeugungssystems (4) erfolgt.

10. Verfahren nach Anspruch 9,
**dadurch gekennzeichnet,**
**dass** die drahtlose Daten-Verbindung (6) über Funk erfolgt.

11. Verfahren nach Anspruch 9
**dadurch gekennzeichnet**
**dass** das separaten Energie Erzeugungssystems mittels eines Thermoelektrischen Generator (4) erfolgt.

12. Verfahren nach Anspruch 11
**dadurch gekennzeichnet,**
**dass** von dem Thermoelektrischen Generator (4) die Temperaturdifferenz zwischen dem von der Prozessenergieenergie (27) erwärmten Bereich und einem von der Prozessenergieenergie abgeschotteten kühlerem Bereich zur Energieerzeugung genutzt wird.
